# EUROPÄISCHE PATENTSCHRIFT

(11) **EP 1 156 810 B1**
(45) Veröffentlichungstag und Bekanntmachung des Hinweises auf die Patenterteilung: **12.05.2004**
(21) Anmeldenummer: 00910715.2
(22) Anmeldetag: 25.02.2000
(51) Int. Cl.: A61K 33/24, A61K 35/78, A61P 19/00

(54) **INJEKTIONSLÖSUNG ENTHALTEND EINE GOLDVERBINDUNG, CALENDULA UND BELLADONNA EXTRAKTE ZUR BEHANDLUNG VON ARTHROSEN**
SOLUTION FOR INJECTION FOR THE TREATMENT OF ARTHROSES
SOLUTION D'INJECTION S'UTILISANT DANS LE TRAITEMENT D'ARTHROSES

(30) Priorität: 26.02.1999 DE 19908441
(43) Veröffentlichungstag der Anmeldung: 28.11.2001
(73) Patentinhaber: Adusumalli, Chakravarty, 94034 Passau (DE)
(72) Erfinder: Adusumalli, Chakravarty, 94034 Passau (DE)
(74) Vertreter: Kaiser, Jürgen, Dr.rer.nat.Dipl.-Chem.
(86) Internationale Anmeldenummer: PCT/EP2000/001602
(87) Internationale Veröffentlichungsnummer: WO 2000/050045

(56) Entgegenhaltungen:
- WO-A-84/04885
- WO-A-97/39355
- WO-A-97/45070
- WO-A-99/49874
- DE-A- 19 544 905
- FR-A- 2 733 419
- GUTTLER H.: "[Anthroposophically oriented medicine]. ANTHROPOSOPHISCH ORIENTIERTE HEILKUNDE." PZ PRISMA, (1998) 5/3 (187-201). , XP000925076
- LIEVRE, M. (1) ET AL: "Controlled study of three ointments for the local management of 2nd and 3rd degree burns." CLINICAL TRIALS AND META-ANALYSIS, (1992) VOL. 28, NO. 1, PP. 9-12. , XP000925064
- DORSCH W: "[Clinical application of extracts of Echinacea purpurea or Echinacea pallida. Critical evaluation of controlled clinical studies]. Klinische Anwendung von Extrakten aus Echinacea purpurea oder Echinacea pallida. Kritische Wertung kontrollierter klinischer Studien." ZEITSCHRIFT FUR ARZTLICHE FORTBILDUNG, (1996 APR) 90 (2) 117-22. REF: 20 , XP000925060

## Beschreibung

Die vorliegende Erfindung betrifft die Behandlung von Arthrosen, vor allem von Hüftgelenks- und Kniegelenksarthrosen.

Insbesondere betrifft die vorliegende Erfindung eine Injektionslösung gemäß Patentanspruch 1, welche eine Mischung aus einer Lösung A einer Goldverbindung sowie einer Lösung B eines Calendula-Extraktes und eines Belladonna-Extraktes umfaßt, die Verwendung einer solchen Injektionslösung zur Behandlung von Arthrosen gemäß Patentanspruch 15, sowie ein Kit umfassend eine Lösung A und eine Lösung B gemäß Patentanspruch 16.

Bei Arthrosen handelt es sich um vorwiegend degenerative Gelenkerkrankungen verschiedenster Ursache, welche den Gelenkknorpel befallen und in der Bevölkerung relativ verbreitet sind.

Arthrosen werden durch einen altersbedingten Verschleiß des Gelenkknorpels begünstigt, so daß sie oft bei älteren Patienten auftreten.

Aber auch übermäßige Belastungen des Knorpelgewebes wie beispielsweise bei Leistungssportlern oder verschiedenen anderen übermäßig beanspruchten Berufsgruppen führen zu einem erhöhten Arthroserisiko.

Schließlich können auch angeborene Störungen, wie z.B. eine angeborene Knorpelminderwertigkeit, Wachstumsstörungen oder entzündliche Gelenkerkrankungen zu Arthrosen führen.

Bisher konnten schwere Arthrosen nur durch einen chirurgischen Eingriff mit einem Austausch des betroffenen Gelenks wie z.B. durch Hüftgelenksoperationen, Kniegelenksoperationen unter Einführung einer Arthroplastik usw. behandelt werden. Diese Operationen sind aber sowohl schmerzhaft als auch zeit- und kostenintensiv.

So betragen beispielsweise die Kosten für eine Hüftarthroplastik in Deutschland durchschnittlich 55.000,- DM, wobei neben der eigentlichen Operation ein längerer Krankenhausaufenthalt und mindestens acht Wochen Rehabilitation notwendig sind. Darüber hinaus besteht ebenfalls die Gefahr von Infektionen sowie die Gefahr, daß sich die eingesetzte Gelenkprothese verschiebt oder lockert oder im Extremfall sogar vom Körper abgestoßen wird.

Daneben existiert eine Vielzahl von Behandlungsverfahren, welche versuchen, eine Besserung unter Erhalt des Gelenks herbeizuführen. Diese umfassen beispielsweise eine Spongiosa-Knochen-Verpflanzung, eine Dekompression und eine Drehosteotomie.

Die Vielzahl der möglichen Behandlungen zeigt, daß bisher noch kein erfolgreiches Verfahren zur Behandlung von Arthrosen gefunden wurde.

Die Druckschrift WO 99/49874 beschreibt pharmazeutische Zusammensetzungen und ein Verfahren zur Behandlung von Gelenkentzündungen und Schmerz, verursacht durch z.B. Arthritis, physisches Trauma, bakterielle oder virale Infektion. In einer Ausführungsform umfaßt das Verfahren die Verabreichung einer Dextran-Formulierung mit bimodalem Molekulargewicht, umfassend 0.2-32 % w/v einer Dextran-Fraktion mit einem durchschnittlichen Molekulargewicht zwischen 30 000 und 110 000 Dalton und 0.2-6 % Dextran mit einem durchschnittlichen Molekulargewicht zwischen 500 und 3 000 Dalton, in das Gelenk eines warmblütigen Tieres.

Die Druckschrift WO 97/45070 betrifft neue injizierbare, wäßrige Suspensionen aus bio-aktivem Glas und Dextranen zur Reperatur von weichem Gewebe oder hartem Knochen von Säugetieren, insbesondere Menschen.

Ausgehend von diesem Stand der Technik ist es Aufgabe der vorliegenden Erfindung, eine neue gelenkerhaltende Behandlungsmöglichkeit für Arthrosen zu schaffen, welche eine hohe Erfolgsquote aufweist, mit geringen Nebenwirkungen und Folgeerkrankungen verbunden ist und außerdem kostengünstig ist.

Die Lösung dieser Aufgabe erfolgt erfindungsgemäß durch eine Injektionslösung gemäß Patentanspruch 1, die Verwendung einer solchen Injektionslösung gemäß Patentanspruch 15, ein Kit gemäß Patentanspruch.

Die erfindungsgemäße Injektionslösung umfaßt eine Mischung aus einer Lösung A einer Goldverbindung sowie einer Lösung B eines Calendula(Ringelblumen)-Extraktes und eines Belladonna(Tollkirschen)-Extraktes.

Mit dieser Injektionslösung können Arthrosen unter Erhalt des Gelenks erfolgreich behandelt werden, die bisher nur durch einen größeren operativen Eingriff zu behandeln waren.

Zur Behandlung von Arthrosen wird die erfindungsgemäße Injektionslösung vorzugsweise wiederholt in die Gelenkhöhle eingespritzt, so daß sie auf den degenerierten Gelenkknorpel einwirken kann.

Die Goldverbindung ist vorzugsweise eine Gold(III)-Verbincung wie beispielsweise die Gold(III)-Verbindung Tetrachlorogold(III)-säure·3 H₂O, welche besonders bevorzugt ist.

Die Injektionslösung kann darüber hinaus einen Hamamelis(Zaubernuß)-Extrakt und/oder einen Arnica(Arnika)-Extrakt und/oder einen Aconitum(Eisenhut)-Extrakt und/oder einen Bellis perennis(Honigbienen)-Extrakt und/oder einen Hypericum(Johanniskraut)-Extrakt und/oder einen Echinacaa angustifolia(Sonnenhut)-Extrakt und/oder einen Echinacea purpurea(roter Sonnenhut)-Extrakt enthalten, wodurch eine weitere Verbesserung des Behandlungserfolges erreicht wird.

Weiterhin kann die Injektionslösung zusätzlich einen Chamomilla(Kamillen)-Extrakt und/oder einen Millefolium(Schaf-garben)-Extrakt enthalten.

Die Injektionslösung kann noch weiter verbessert werden, wenn sie zusätzlich noch einen Symphytum(Beinwellwurzel)-Extrakt und/oder Mercuris solubilis Hahnemanni (flüssiges Quecksilber, hergestellt nach Hahnemann) und/oder Hepar sulfuris (Schwefelleber) enthält.

Die oben aufgeführten zusätzlichen Bestandteile sind dabei vorzugsweise in der Lösung A enthalten.

Als weitere Bestandteile der Injektionslösung, welche vorzugsweise zusätzlich in der Lösung B enthalten sind, können ein Extrakt aus Fruct. Symphoricarpi (Schneebeere) und/oder ein Extrakt aus Herb. Euphorbiae cyp. (Wolfsmichgewächsen) enthalten sein.

Die Pflanzenextrakte werden vorzugsweise als D1- bis D6-Verdünnungen nach Hahnemann eingesetzt, um die Lösungen A und B und anschließend die Injektionslösung zuzubereiten.

Eine D1-Verdünnung nach Hahnemann wird beispielsweise dadurch erhalten, daß ein Auspreßsaft in einem Verhältnis von 1:10 mit 45% Alkohol verdünnt wird. Die weiteren Verdünnungen D2, D3 usw. können dann durch eine erneute 1:10-Verdünnung mit Alkohol erhalten werden; d.h. um eine D2-Verdünnung herzustellen wird ein Teil der D1-Verdünnung mit 9 Teilen Alkohol verdünnt.

Eine bevorzugte Injektionslösung ist eine Injektionslösung gemäß Patentanspruch 7, in welcher die Lösung A zwischen 0,01 und 10 µl/ml Arnica D2, Calendula D2, Chamomilla D3, Symphytum D6, Millefolium D3, Belladonna D2, Aconitum D2, Bellis perennis D2, Hypericum D2, Echinacea angustifolia D2, Echinacea purpurea D2, Hamamelis D1, Mercuris solubilis Hahnemanni D6 und Hepar sulfuris D6 enthält, und die Lösung B eine Gold(III)-Verbindung, Fruct. Symphoricarpi und Herb. Euphorbiae cyp. enthält.

Eine besonders bevorzugte Injektionslösung ist eine Injektionslösung nach Patentanspruch 9, in welcher die Lösung A je 0,1 bis 10 µl/ml Arnica D2, Calendula D2, Chamomilla D3, Symphytum D6, Millefolium D3 und Belladonna D2, 0,05 bis 5 µl/ml Aconitum D2, 0,05 bis 5 µl/ml Bellis perennis D2, 0,05 bis 5 µl/ml Hypericum D2, 0,02 bis 2 µl/ml Echinacea angustifolia D2, 0,02 bis 2 µl/ml Echinacea purpurea D2, 0,01 bis 1 µl/ml Hamamelis D1, 0,05 bis 5 mg/ml Mercuris solubilis Hahnemanni D6, 0,1 bis 10 µl/ml Hepar sulfuris D6; NaCl in physiologischer Konzentration und Wasser enthält; und die Lösung B 0,001 bis 0,1 mg/ml Gold in Form einer Gold(III)-Verbindung, einen wäßrigen Extrakt (10:1 bis 1:10) aus Fruct. Symphoricarpi (0,2 bis 20 mg/ml) und Herb. Euphorbiae cyp. (0,1 bis 10 mg/ml) sowie NaCl in physiologischer Konzentration und Wasser enthält.

Die am meisten bevorzugte Injektionslösung ist die Injektionslösung nach Patentanspruch 10, in welcher die Lösung A je ca. 1 µl/ml Arnica D2, Calendula D2, Chamomilla D3, Symphytum D6, Millefolium D3 und Belladonna D2, ca. 0,6 µl/ml Aconitum D2, ca. 0,5 µl/ml Bellis perennis D2, ca. 0,3 µl/ml Hypericum D2, ca. 0,25 µl/ml Echinacea angustifolia D2, ca. 0,25 µl/ml Echinacea purpurea D2, ca. 0,1 µl/ml Hamamelis D1, ca. 0,5 mg/ml Mercuris solubilis Hahnemanni D6, ca. 1 µl/ml Hepar sulfuris D6; NaCl in physiologischer Konzentration und Wasser enthält; und die Lösung B ca. 0,01 mg/ml Gold in Form einer Gold(III)-Verbindung, einen wäßrigen Extrakt (ca. 1:1) aus Fruct. Symphoricarpi (ca. 2 mg/ml) und Herb. Euphorbiae cyp. (ca. 1 mg/ml) sowie NaCl in physiologischer Konzentration und Wasser enthält.

Eine solche Injektionslösung kann beispielsweise durch ein Mischen der im Handel erhältlichen Lösung Traumeel® S der Firma Biologische Heilmittel Heel GmbH aus Baden-Baden und der ebenfalls im Handel erhältlichen Lösung Cefossin® H der Firma Cefak Arzneimittel aus Kempten in einem Volumenverhältnis von ca. 1:1 erhalten werden.

Die Injektionslösung ist gewöhnlich mit einer physiologischen Salzlösung, beispielsweise einer physiologischen Kochsalzlösung, ergänzt und sie basiert normalerweise auf Wasser.

Ein wesentlicher Punkt der vorliegenden Erfindung ist der, daß die Injektionslösung besonders wirksam ist, wenn sie erst kurz vor der Verabreichung durch ein Mischen der jeweiligen Lösungen A und B hergestellt wird. Je länger die Zeitspanne zwischen dem Mischen und der Verabreichung ist, desto weniger wirksam ist die Injektionslösung.

Die Lösungen A und B werden dabei vorzugsweise in einem Volumenverhältnis von 1:10 bis 10:1, insbesondere von 1:2 bis 2:1 und besonders bevorzugt von ca. 1:1 gemischt.

Besonders bevorzugt liegt die erfindungsgemäße Injektionslösung als eine intraartikuläre Injektionslösung vor.

Ein weiterer wesentlicher Aspekt der vorliegenden Erfindung ist die Verwendung der erfindungsgemäßen Injektionslösung zur Behandlung von Arthrosen, insbesondere von Knie- und Hüftgelenksarthrosen, durch intraartikuläre Injektion.

Im Gegensatz zu den üblichen Behandlungsverfahren führt die erfindungsgemäße Verwendung zu einem gelenkerhaltenden Verfahren (Außenseiterverfahren) und führt zu sehr guten Heilergebnissen, so daß eine operative Behandlung der Arthrosen überflüssig wird.

Die vorliegende Erfindung stellt weiterhin ein Kit zur Verfügung, welches eine Lösung A sowie eine Lösung B umfaßt. Dieses Kit bietet die Möglichkeit, die erfindungsgemäße Injektionslösung auf einfache und schnelle Weise unmittelbar vor der Anwendung durch ein Mischen der beiden Lösungen herzustellen.

Das Kit kann dabei jeweils eine oder auch mehrere sterile Ampullen mit den verschiedenen Lösungen enthalten.

Das Kit kann weiterhin eine Dextranlösung umfassen, welche zur Spülung der Gelenke vor und/oder nach der Injektion der erfindungsgemäßen Injektionslösung eingesetzt werden kann. Eine solche Dextranlösung ist z.B. die im Handel erhältliche Makrodex®-Lösung von der Firma Schiwa GmbH aus Glandorf.

Die Verwendung einer solchen Dextran-Lösung zur Spülung der Gelenke trägt ebenfalls zu dem Erfolg der Behandlung mit der Injektionslösung gemäß der Erfindung bei.

Weitere Vorteile und Merkmale der vorliegenden Erfindung ergeben sich anhand der Beschreibung von Ausführungsbeispielen sowie anhand der Zeichnung, in welcher:
- Fig. 1: eine Videoröntgenaufnahme eines Hüftgelenks nach 10-maligem Einspritzen der erfindungsgemäßen Injektionslösung, jedoch vor der Ausspülung, zeigt,
- Fig. 2: eine Videoröntgenaufnahme desselben Hüftgelenks bei der Behandlung zeigt, wobei sich eine Kanüle in dem Hüftgelenkspalt befindet, durch welche das Gelenk ausgespült wird,
- Fig. 3: eine Videoröntgenaufnahme desselben Hüftgelenks nach der Ausspülung zeigt,
- Fig. 4: eine Videoröntgenaufnahme eines Kniegelenks nach 10-maliger Behandlung mit der erfindungsgemäßen Injektionslösung, jedoch vor der Ausspülung, zeigt,
- Fig. 5: eine Videoröntgenaufnahme desselben Kniegelenks bei der Behandlung zeigt, wobei sich zwei Kanülen im Kniegelenkspalt befinden, durch welche das Gelenk ausgespült wird, und
- Fig. 6: eine Videoröntgenaufnahme desselben Kniegelenks nach der Ausspülung zeigt.

Im folgenden wird eine Studie beschrieben, welche der Erfinder der vorliegenden Anmeldung mit 127 Arthrosepatienten durchgeführt hat.

Zwischen Dezember 1990 und Juli 1997 wurden unter Überwachung des gegenwärtigen Erfinders 127 Patienten (168 Hüften) mit einer Injektionslösung behandelt, welche wie folgt hergestellt wurde: 2,5 ml einer Lösung A, welche im folgenden als Cartilase A bezeichnet wird, und 2,5 ml einer Lösung B (bezeichnet als Cartilase B) wurden jeweils kurz vor der Injektion gemischt, um die Injektionslösung herzustellen. Die Zusammensetzungen der beiden Lösungen waren wie folgt:

| | | |
|---|---|---|
| Cartilase A | Arzneilich wirksame Bestandteile: | |
| (1 ml) | Gold (in Form von 0,0209 mg Tetra chlorogold(III)-säure·3 H₂O) | 0,01 mg |
| | Extr. aquos. (1:1) aus Fruct. Symphoricarpi | 2 mg |
| | Herb. Euphorbiae cyp. | 1 mg |
| | Sonstige Bestandteile: | |
| | Natriumchlorid und Wasser. | |
| | | |
| Cartilase B | Arnica D2 | 2,2 µl |
| (2,2 ml) | Calendula D2 | 2,2 µl |
| | Chamomilla D3 | 2,2 µl |
| | Symphytum D6 | 2,2 µl |
| | Millefolium D3 | 2,2 µl |
| | Belladonna D2 | 2,2 µl |
| | | |
| | Aconitum D2 | 1,32 µl |
| | Bellis perennis D2 | 1,1 µl |
| | Hypericum D2 | 0,66 µl |
| | Echinacea angustifolia D2 | 0,55 µl |
| | Echinacea purpurea D2 | 0,55 µl |
| | Hamamelis D1 | 0,22 µl |
| | Mercuris solub. Hahnemanni D6 | 1,1 mg |
| | Hepar sulfuris D6 | 2,2 µl |
| | Hilfsstoffe: Natriumchlorid und Wasser | |

Das Alter der behandelten Patienten betrug zwischen 31 und 76 Jahren. Alle Patienten hatten die Diagnose einer Osteoarthritis und Verletzungsknochennekrose. Vor der Behandlung hatten alle Patienten starke Schmerzen beim Tragen schwerer Lasten, Schmerzen in der Nacht und sogar Schmerzen in Ruhe ohne Belastung.

Die Röntgenaufnahmen vor der Behandlung zeigten eine Osteoarthritis und eine Nekrose mit Knochensequesterbildung mit einem unterschiedlichen Grad einer Schädigung des Femurkopfes in 15% der Fälle. In der Studie wurde der Gelenkzwischenraum bzw. die Gelenkspalte vor und nach der Behandlung in Millimetern gemessen. Die Breite des Gelenkzwischenraums bei der letzten Untersuchung wurde dann am Ende mit früheren Röntgenaufnahmen verglichen.

Die Behandlung erfolgte wie im folgenden beschrieben:

Den Patienten wurde wöchentlich über 10 Wochen hinweg eine intraartikuläre Injektion mit 5 ml der obigen Injektionslösung verabreicht, welche so auf den degenerierten Gelenkknorpel einwirken und diesen lösen konnte. Nach dem Abschluß dieser Behandlung wurde der Patient betäubt, und das Hüftgelenk wurde mit Hilfe eines 25 kg-Gewichts gestreckt. Dann wurde eine Kanüle in die Gelenkspalte eingeführt, das Hüftgelenk wurde mit einer hochmolekularen Flüssigkeit in Form einer Makrodex®-Lösung gefüllt, um die im Gelenkzwischenraum befindlichen Adhäsionen bzw. den gelösten degenerierten Gelenkknorpel herauszuspülen und zu entfernen, und anschließend wurde erneut die oben angeführte Injektionslösung eingespritzt. Nach dieser Reinigung des Gelenks wurde ein Kontrastmittel bzw. ein Farbstoff zur Röntgenuntersuchung injiziert.

Im Gegensatz zu der herkömmlichen Hüftgelenksoperation konnte der Patient bei dieser Behandlung nach 24 bis 48 Stunden aus dem Krankenhaus entlassen werden.

Nach der Behandlung wurden die Patienten für 24 Monate weiter überwacht.

Nach diesen 24 Monaten zeigten sich bei 85% der Patienten sehr gute Ergebnisse und bei weiteren 7% zufriedenstellende Ergebnisse, was bedeutete, daß diese Patienten ohne Schmerzen laufen konnten. Nur 8% der Patienten wiesen keine zufriedenstellenden Ergebnisse auf und diesen wurde eine herkömmliche Hüftgelenksplastik implantiert.

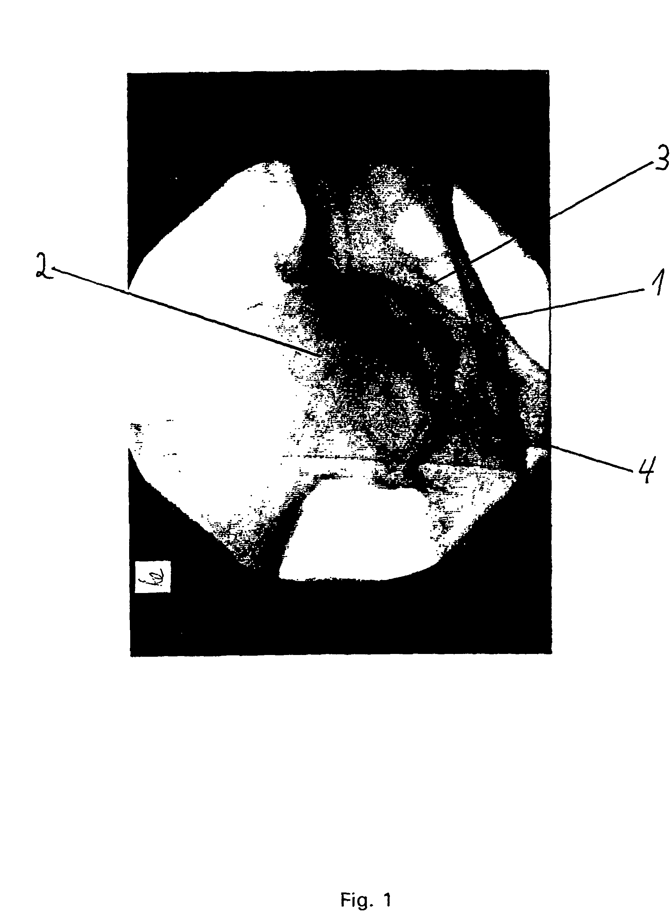

Die Figuren 1, 2 und 3 veranschaulichen die Ergebnisse einer Behandlung eines Hüftgelenks mit einer erfindungsgemäßen Injektionslösung.

Wie man in Fig. 1 sieht, ist der Gelenkspalt 1 des Hüftgelenks, welcher sich zwischen dem Femurkopf 2 und der gegenüberliegenden Gelenkpfanne 3 befindet, mit degeneriertem Gelenkknorpelmaterial 4 zugesetzt.

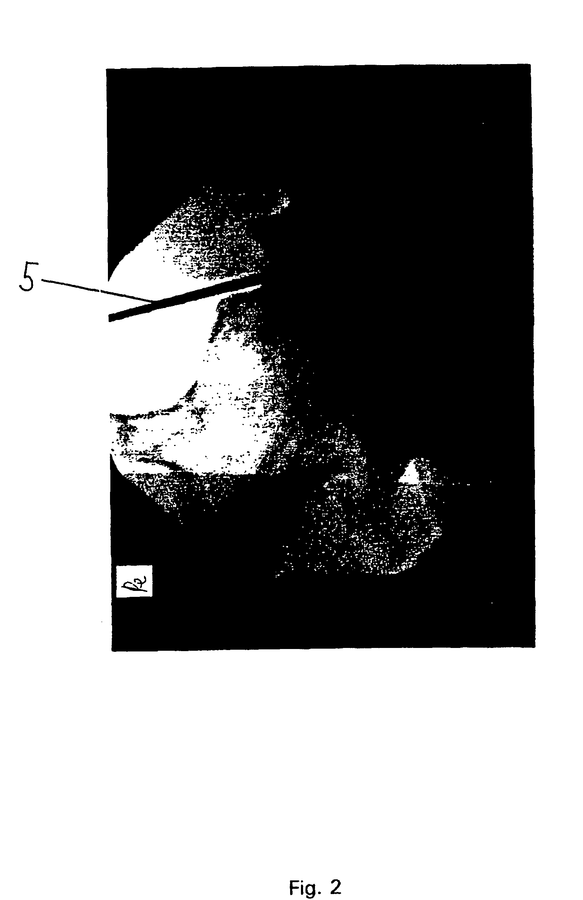

Fig. 2 zeigt dasselbe Hüftgelenk während des Ausspülens der Injektionslösung, wobei eine Kanüle 5 in dem Gelenkspalt 1 sichtbar ist. Durch die Kanüle 5 wurde der Gelenkspalt 1 sechs- bis achtmal mit je 20 ml einer hochmolekularen Dextranlösung ausgespült, d.h. die Dextranlösung wurde in den Gelenkspalt 1 gespritzt und abgesaugt, um das Gelenk zu reinigen.

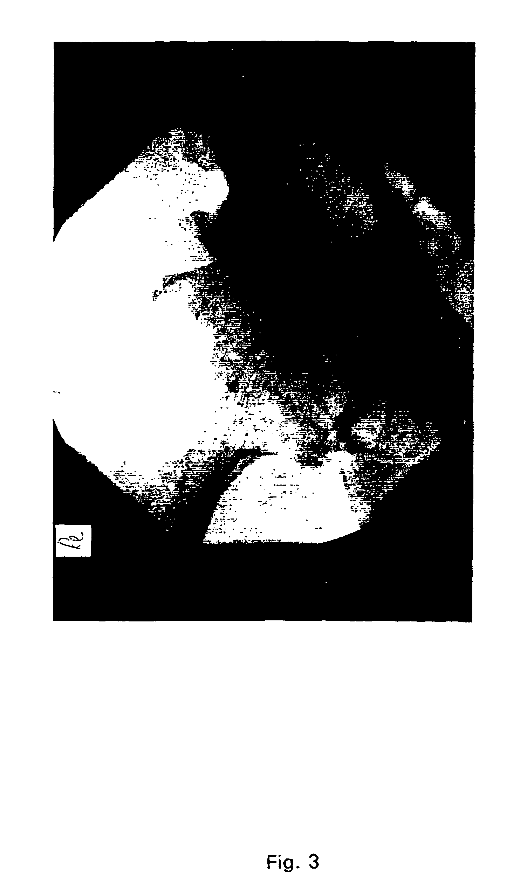

Wie man in Fig. 3 erkennen kann, ist nach Abschluß der Behandlung eine deutliche Verbesserung der Situation eingetreten, wobei ein Großteil des degenerierten Knorpelmaterials aus dem Gelenkspalt 1 entfernt wurde.

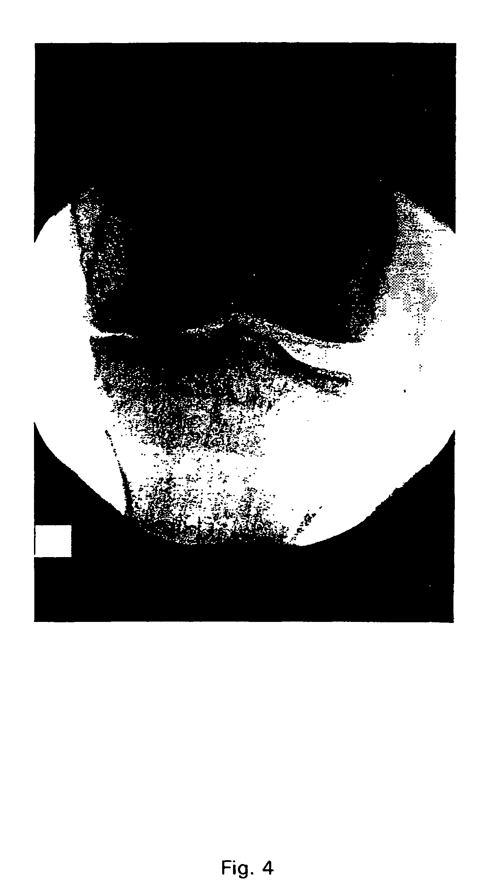

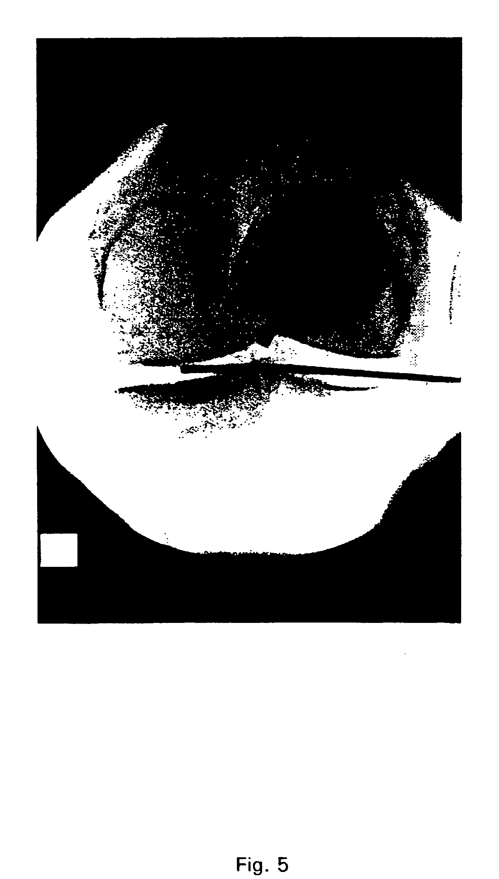

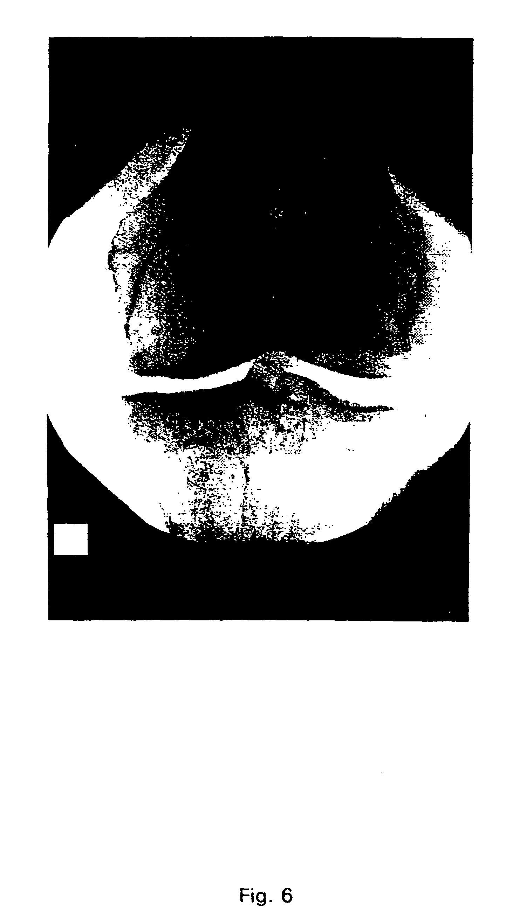

Dasselbe Ergebnis ist ebenfalls aus den Figuren 4 bis 6 ersichtlich, welche entsprechend ein Kniegelenk bei bzw. nach der Behandlung mit der erfindungsgemäßen Injektionslösung zeigen.

Auch in diesem Fall ist der Gelenkspalt des Kniegelenks nach der Behandlung und Ausspülung von dem größten Teil des degenerierten Gelenkknorpelmaterials befreit.

Diese Untersuchungen machen deutlich, daß die Behandlung mit der erfindungsgemäßen Injektionslösung eine erfolgreiche alternative Behandlungsmöglichkeit darstellt, die das Fortschreiten der Krankheit in 85% der Fälle aufhalten kann.

## Patentansprüche

1. Injektionslösung, umfassend eine Mischung aus einer Lösung A einer Goldverbindung sowie einer Lösung B eines Calendula-Extraktes und eines Belladonna-Extraktes.

2. Injektionslösung nach Anspruch 1, **dadurch gekennzeichnet, daß** die Goldverbindung eine Gold(III)-Verbindung ist.

3. Injektionslösung nach Anspruch 1 oder 2, **dadurch gekennzeichnet, daß** die Lösung A und/oder die Lösung B weiterhin einen Hamamelis-Extrakt und/oder einen Arnica-Extrakt und/oder einen Aconitum-Extrakt und/oder einen Bellis perennis-Extrakt und/oder einen Hypericum-Extrakt und/oder einen Echinacea angustifolia-Extrakt und/oder einen Echinacea purpurea-Extrakt enthält.

4. Injektionslösung nach einem der Ansprüche 1 bis 3, **dadurch gekennzeichnet, daß** die Lösung A und/oder die Lösung B weiterhin einen Chamomilla-Extrakt und/oder einen Millefolium-Extrakt enthält.

5. Injektionslösung nach einem der Ansprüche 1 bis 4, **dadurch gekennzeichnet, daß** die Lösung A und/oder die Lösung B weiterhin einen Symphytum-Extrakt und/oder Mercuris solubilis Hahnemanni und/oder Hepar sulfuris enthält.

6. Injektionslösung nach einem der Ansprüche 1 bis 5, **dadurch gekennzeichnet, daß** die Lösung B und/oder die Lösung A weiterhin einen Extrakt aus Fruct. Symphoricarpi und/oder einen Extrakt aus Herb. Euphorbiae cyp. enthält.

7. Injektionslösung nach einem der Ansprüche 1 bis 6, **dadurch gekennzeichnet, daß** die Lösung A zwischen 0,01 und 10 µl/ml Arnica D2, Calendula D2, Chamomilla D3, Symphytum D6, Millefolium D3, Belladonna D2, Aconitum D2, Bellis perennis D2, Hypericum D2, Echinacea angustifolia D2, Echinacea purpurea D2, Hamamelis D1, Mercuris solubilis Hahnemanni D6 und Hepar sulfuris D6 enthält, und die Lösung B eine Gold(III)-Verbindung, Fruct. Symphoricarpi und Herb. Euphorbiae cyp. enthält.

8. Injektionslösung nach einem der Ansprüche 1 bis 7, **dadurch gekennzeichnet, daß** diese weiterhin NaCl, insbesondere in einer physiologischen Konzentration, und Wasser enthält.

9. Injektionslösung nach einem der Ansprüche 1 bis 8, **dadurch gekennzeichnet, daß** die Lösung A je 0,1 bis 10 µl/ml Arnica D2, Calendula D2, Chamomilla D3, Symphytum D6, Millefolium D3 und Belladonna D2, 0,05 bis 5 µl/ml Aconitum D2, 0,05 bis 5 µl/ml Bellis perennis D2, 0,05 bis 5 µl/ml Hypericum D2, 0,02 bis 2 µl/ml Echinacea angustifolia D2, 0,02 bis 2 µl/ml Echinacea purpurea D2, 0,01 bis 1 µl/ml Hamamelis D1, 0,05 bis 5 mg/ml Mercuris solubilis Hahnemanni D6, 0,1 bis 10 µl/ml Hepar sulfuris D6; NaCl in physiologischer Konzentration und Wasser enthält; und die Lösung B 0, 001 bis 0,1 mg/ml Gold in Form einer Gold(III)-Verbindung, einen wäßrigen Extrakt (10:1 bis 1:10) aus Fruct. Symphoricarpi (0,2 bis 20 mg/ml) und Herb. Euphorbiae cyp. (0,1 bis 10 mg/ml) sowie NaCl in physiologischer Konzentration und Wasser enthält.

10. Injektionslösung nach einem der Ansprüche 1 bis 9, **dadurch gekennzeichnet, daß** die Lösung A je ca. 1 µ l/ml Arnica D2, Calendula D2, Chamomilla D3, Symphytum D6, Millefolium D3 und Belladonna D2, ca. 0,6 µl/ml Aconitum D2, ca. 0,5 µl/ml Bellis perennis D2, ca. 0,3 µl/ml Hypericum D2, ca. 0,25 µl/ml Echinacea angustifolia D2, ca. 0,25 µl/ml Echinacea purpurea D2, ca. 0,1 µl/ml Hamamelis D1, ca. 0,5 mg/ml Mercuris solubilis Hahnemanni D6, ca. 1 µl/ml Hepar sulfuris D6; NaCl in physiologischer Konzentration und Wasser enthält; und die Lösung B ca. 0,01 mg/ml Gold in Form einer Gold(III)-Verbindung, einen wäßrigen Extrakt (ca. 1:1) aus Fruct. Symphoricarpi (ca. 2 mg/ml) und Herb. Euphorbiae cyp. (ca. 1 mg/ml) sowie NaCl in physiologischer Konzentration und Wasser enthält.

11. Injektionslösung nach einem der Ansprüche 1 bis 10, **dadurch gekennzeichnet, daß** die Gold(III)-Verbindung Tetrachlorogold(III)-säure·3 H₂O ist.

12. Injektionslösung nach einem der Ansprüche 1 bis 11, **dadurch gekennzeichnet, daß** sie unmittelbar vor der Injektion durch ein Mischen der Lösung A mit der Lösung B frisch hergestellt wird.

13. Injektionslösung nach Anspruch 12, **dadurch gekennzeichnet, daß** die Lösungen A und B in einem Volumenverhältnis von 1:10 bis 10:1, insbesondere von 1:2 bis 2:1 und besonders bevorzugt von ca. 1:1 gemischt werden.

14. Injektionslösung nach einem der Ansprüche 1 bis 13, **dadurch gekennzeichnet, daß** die Injektionslösung eine intraartikuläre Injektionslösung ist.

15. Verwendung der Injektionslösung nach einem der Ansprüche 1 bis 14 zur Herstellung eines Medikaments zur Behandlung von Arthrosen, insbesondere von Knie- und Hüftgelenksarthrosen, durch intraartikuläre Injektion.

16. Kit umfassend eine Lösung A, wie in einem der Ansprüche 1 bis 11 definiert, sowie eine Lösung B, wie in einem der Ansprüche 1 bis 11 definiert.

17. Kit nach Anspruch 16, **dadurch gekennzeichnet, daß** es weiterhin eine Dextranlösung umfaßt.

## Claims

1. An injection solution including a mixture of a solution A of a gold compound and of a solution B of a *Calendula* extract and of a *Belladonna* extract.

2. An injection solution in accordance with claim 1, **characterised in that** said gold compound is a gold(III) compound.

3. An injection solution in accordance with claim 1 or claim 2, **characterised in that** said solution A and/or solution B moreover contains a *Hamamelis* extract and/or an *Arnica* extract and/or an *Aconitum* extract and/or a *Bellis perennis* extract and/or a *Hypericum* extract and/or an *Echinacea angustifolia* extract and/or an *Echinacea purpurea* extract.

4. An injection solution in accordance with any one of claims 1 to 3, **characterised in that** said solution A and/or solution B moreover contains a *Chamomilla* extract and/or a *Millefolium* extract.

5. An injection solution in accordance with any one of claims 1 to 4, **characterised in that** said solution A and/or solution B moreover contains a *Symphytum* extract and/or *Mercuris solubilis Hahnemanni* and/or *Hepar sulfuris.*

6. An injection solution in accordance with any one of claims 1 to 5, **characterised in that** said solution B and/or solution A moreover contains an extract from *Fruct*. *Symphoricarpi* and/or an extract from *Herb. Euphorbiae cyp*..

7. An injection solution in accordance with any one of claims 1 to 6, **characterised in that** said solution A contains between 0.01 and 10 µl/ml of *Arnica* D2, *Calendula* D2, *Chamomilla* D3, *Symphytum* D6, *Millefolium* D3, *Belladonna* D2, *Aconitum* D2, *Bellis perennis* D2, *Hypericum* D2, *Echinacea angustifolia* D2, *Echinacea purpurea* D2, *Hamamelis* D1, *Mercuris solubilis Hahnemanni* D6 and *Hepar sulfuris* D6, and said solution B contains a gold(III) compound, *Fruct. Symphoricarpi* and *Herb. Euphorbiae cyp*..

8. An injection solution in accordance with any one of claims 1 to 7, **characterised in that** said solution moreover contains NaCl, in particular in a physiological concentration, and water.

9. An injection solution in accordance with any one of claims 1 to 8, **characterised in that** said solution A contains 0.1 to 10 µl/ml each of *Arnica* D2, *Calendula* D2, *Chamomilla* D3, *Symphytum* D6, *Millefolium* D3 and *Belladonna* D2, 0.05 to 5 µl/ml of *Aconitum* D2, 0.05 to 5 µl/ml of *Bellis perennis* D2, 0.05 to 5 µl/ml of *Hypericum* D2, 0.02 to 2 µl/ml of *Echinacea angustifolia* D2, 0.02 to 2 µl/ml of *Echinacea purpurea* D2, 0.01 to 1 µl/ml of *Hamamelis* D1, 0.05 to 5 mg/ml of *Mercuris solubilis Hahnemanni* D6, 0.1 to 10 µl/ml of *Hepar sulfuris* D6; NaCl in physiological concentration, and water; and said solution B contains 0.001 to 0.1 mg/ml of gold in the form of a gold(III) compound, an aqueous extract (10:1 to 1:10) from *Fruct. Symphoricarpi* (0.2 to 20 mg/ml) and Herb. *Euphorbiae cyp*. (0.1 to 10 mg/ml), as well as NaCl in physiological concentration, and water.

10. An injection solution in accordance with any one of claims 1 to 9, **characterised in that** said solution A contains approx. 1 µl/ml each of *Arnica* D2, *Calendula* D2, *Chamomilla* D3, *Symphytum* D6, *Millefolium* D3 and *Belladonna* D2, approx. 0.6 µl/ml of *Aconitum* D2, approx. 0.5 µl/ml of *Bellis perennis* D2, approx. 0.3 µl/ml of *Hypericum* D2, approx. 0.25 µl/ml of *Echinacea angustifolia* D2, approx. 0.25 µl/ml of *Echinacea purpurea* D2, approx. 0.1 µl/ml of *Hamamelis* D1, approx. 0.5 mg/ml of *Mercuris solubilis Hahnemanni* D6, *approx.* 1 µl/ml of *Hepar sulfuris* D6; NaCl in physiological concentration, and water; and said solution B contains approx. 0.01 mg/ml of gold in the form of a gold(III) compound, an aqueous extract (approx. 1:1) from *Fruct. Symphoricarpi* (approx. 2 mg/ml) and *Herb. Euphorbiae cyp*. (approx. 1 mg/ml), as well as NaCl in physiological concentration and water.

11. An injection solution in accordance with any one of claims 1 to 10, **characterised in that** said gold(III) compound is tetrachloroauric(III) acid·3 H₂O.

12. An injection solution in accordance with any one of claims 1 to 11, **characterised in that** it is freshly prepared immediately prior to injection by mixing solution A with solution B.

13. An injection solution in accordance with claim 12, **characterised in that** said solutions A and B are mixed in a volume ratio of 1:10 to 10:1, in particular of 1:2 to 2:1, and in a particularly preferred manner of approx. 1:1.

14. An injection solution in accordance with any one of claims 1 to 13, **characterised in that** said injection solution is an intra-articular injection solution.

15. Use of said injection solution in accordance with any one of claims 1 to 14 for producing a medicament for the treatment of arthroses, in particular of arthroses of the knee and hip joint, by intra-articular injection.

16. A kit including a solution A as defined in any one of claims 1 to 11, as well as a solution B as defined in any one of claims 1 to 11.

17. A kit in accordance with claim 16, **characterised in that** it furthermore includes a dextrane solution.

## Revendications

1. Solution d'injection comportant un mélange constitué d'une solution A d'un composé d'or ainsi que d'une solution B d'un extrait de Calendula et d'un extrait de Belladona.

2. Solution d'injection selon la revendication 1, **caractérisée en ce que** le composé d'or est un composé d'or (III).

3. Solution d'injection selon la revendication 1 ou 2, **caractérisée en ce que** la solution A et/ou la solution B contient en outre un extrait d'Hamamelis et/ou un extrait d'Arnica et/ou un extrait d'Aconitum et/ou un extrait de Bellis perennis et/ou un extrait d'Hypericum et/ou un extrait d'Echinacea angustifolia et/ou un extrait Echinacea purpurea.

4. Solution d'injection selon l'une quelconque des revendications 1 à 3, **caractérisée en ce que** la solution A et/ou la solution B contient en outre un extrait de Chamomilla et/ou un extrait de Millefolium.

5. Solution d'injection selon l'une quelconque des revendications 1 à 4, **caractérisée en ce que** la solution A et/ou la solution B contient en outre un extrait de Symphytum et/ou de Mercuris solubilis Hahnemanni et/ou de Hepar sulfuris.

6. Solution d'injection selon l'une quelconque des revendications 1 à 5, **caractérisée en ce que** la solution B et/ou la solution A contient en outre un extrait de Fruct. Symphoricarpi et/ou un extrait de Herb. Euphorbiae cyp.

7. Solution d'injection selon l'une quelconque des revendications 1 à 6, **caractérisée en ce que** la solution A contient entre 0,01 et 10 µl/ml d'Arnica D2, de Calendula D2, de Chamomilla D3, de Symphytum D6, de Millefolium D3, de Belladonna D2, d'Aconitum D2, de Bellis perennis D2, d'Hypericum D2, d'Echinacea angustifolia D2, d'Echinacea purpurea D2, d'Hamamelis D1, de Mercuris solubilis Hahnemanni D6 et d'Hepar sulfuris D6 et la solution B un composé d'or (III), du Fruct. Symphoricarpi et de l'Herb. Euphorbiae cyp.

8. Solution d'injection selon l'une quelconque des revendications 1 à 7, **caractérisée en ce que** celle-ci contient en outre du NaCl, en particulier dans une concentration physiologique, et de l'eau.

9. Solution d'injection selon l'une quelconque des revendications 1 à 8, **caractérisée en ce que** la solution A contient 0,1 à 10 µl/ml de chacune des substances Arnica D2, Calendula D2, Chamomilla D3, Symphytum D6, Millefolium D3 et Belladonna D2, 0,5 à 5 µl/ml d'Aconitum D2, 0,05 à 5 µl/ml de Bellis perennis D2, 0,05 à 5 µl/ml d'Hypericum D2, 0,02 à 2 µl/ml d'Echinacea angustifolia D2, 0,02 à 2 µl/ml d'Echinacea purpurea D2, 0,01 à 1 µl/ml d'Hamamelis D1, 0,05 à 5 mg/ml de Mercuris solubilis Hahnemanni D6, 0,1 à 10 µl/ml d'Hepar sulfuris D6 ; du NaCl en concentration physiologique et de l'eau ; et la solution B 0,001 à 0,1 mg/ml d'or sous forme d'un composé d'or (III), un extrait aqueux (10:1 à 1:10) de Fruct. Symphoricarpi (0,2 à 20 mg/ml) et d'Herb. Euphorbiae cyp. (0,1 à 10 mg/ml) ainsi que du NaCl en concentration physiologique et de l'eau.

10. Solution d'injection selon l'une quelconque des revendications 1 à 9, **caractérisée en ce que** la solution A contient environ 1 µl/ml de chacune des substances Arnica D2, Calendula D2, Chamomilla D3, Symphytum D6, Millefolium D3 et Belladonna D2, environ 0,6 µl/ml d'Aconitum D2, environ 0,5 µl/ml de Bellis perennis D2, environ 0,3 µl/ml d'Hypericum D2, environ 0,25 µl/ml d'Echinacea angustifolia D2, environ 0,25 µl/ml d'Echinacea purpurea D2, environ 0,1 µl/ml d'Hamamelis D1, environ 0,5 mg/ml de Mercuris solubilis Hahnemanni D6, environ 1 µl/ml d'Hepar sulfuris D6 ; du NaCl en concentration physiologique et de l'eau ; et la solution B environ 0,01 mg/ml d'or sous forme d'un composé d'or (III), un extrait aqueux (environ 1:1) de Fruct. Symphoricarpi (environ 2 mg/ml) et d'Herb. Euphorbiae cyp. (environ 1 mg/ml) ainsi que du NaCl en concentration physiologique et de l'eau.

11. Solution d'injection selon l'une quelconque des revendications 1 à 10, **caractérisée en ce que** le composé d'or (III) est de l'acide tétrachloroaurique (III) · 3H₂O.

12. Solution d'injection selon l'une quelconque des revendications 1 à 11, **caractérisée en ce qu'**elle est fraîchement préparée immédiatement avant l'injection en mélangeant la solution A avec la solution B.

13. Solution d'injection selon la revendication 12, **caractérisée en ce que** les solutions A et B sont mélangées dans un rapport volumique de 1:10 à 10:1, en particulier de 1:2 à 2:1 et de manière particulièrement préférée d'environ 1:1.

14. Solution d'injection selon l'une quelconque des revendications 1 à 13, **caractérisée en ce que** la solution d'injection est une solution d'injection intra-articulaire.

15. Utilisation de la solution d'injection selon l'une quelconque des revendications 1 à 14 pour fabriquer un médicament destiné à traiter l'arthrose, en particulier les arthroses du genou et de la hanche, par injection intra-articulaire.

16. Kit comportant une solution A, telle que définie dans l'une quelconque des revendications 1 à 11, ainsi qu'une solution B, telle que définie dans l'une quelconque des revendications 1 à 11.

17. Kit selon la revendication 16, **caractérisé en ce qu'**il comporte en outre une solution de dextrane.
